# EUROPÄISCHE PATENTANMELDUNG

(11) **EP 1 306 791 A2**
(43) Veröffentlichungstag der Anmeldung: **02.05.2003**
(21) Anmeldenummer: 02021684.2
(22) Anmeldetag: 27.09.2002
(51) Int. Cl.: G06F 19/00

(54) **Vorrichtung zur Beratung von Ärzten unter lizenzkontrollierten Bedingungen**

(30) Priorität: 25.10.2001 DE 10152735
(71) Anmelder: SIEMENS AKTIENGESELLSCHAFT, 80333 München (DE)
(72) Erfinder: Kuth, Rainer, 91074 Herzogenaurach (DE); Zindel, Christoph, Dr., 91080 Uttenreuth (DE)

(57) **Zusammenfassung**

Vorrichtung zur Zugänglichmachung von Expertenwissen für einen Nutzer (Arzt) eines medizinischen Untersuchungsgerätes, wobei ein Übertragungssystem eines Handhabungsprocederes in Form von Pulssequenzen, Protokollen, Parametersätzen od. dgl. für das Untersuchungsgerät, abgestimmt auf konkrete medizinische Diagnosen des anfragenden Nutzers, mit einem Lizenzabrechnungssystem gekoppelt ist, das dem das Handhabungsprocedere erstellenden Experten eine Lizenzvergütung zukommen lässt, sowie mit einer Sperrvorrichtung, die eine erweiterte Nutzung über die vereinbarte und bezahlte Nutzung hinaus verhindert.

## Beschreibung

Die Erfindung bezieht sich auf ein System zur Zugänglichmachung von Expertenwissen für einen Nutzer (Arzt) eines medizinischen Untersuchungsgerätes.

Die Komplexität bei der Nutzung medizinischer Untersuchungsgeräte, insbesondere bei radiologischen Untersuchungen, nimmt laufend zu. Immer mehr Diagnosen erfordern ein profundes Fachwissen bezüglich sehr selten auftretender Erkrankungen. Daher besteht bei vielen Ärzten, speziell im angesprochenen Fall bei vielen Radiologen, Bedarf an einer fachlichen Beratung und Unterstützung durch hochspezialisierte Fachkollegen. Letztere kennen klinische Procederes oder sind in der Lage diese zu entwickeln, welche aus speziellen Pulssequenzen, Protokollen Parameteransätzen od. dgl. entstehen können, die vor unlizensierter Benutzung geschützt werden müssen, um einer Kommerzialisierung zugänglich gemacht werden zu können.

Aus der DE 196 37 219 A1 ist bereits eine Vorrichtung zum Erfassen, Bearbeiten und Auswerten medizinischer Daten vorbekannt, bei der zur Unterstützung eines lokal niedergelassenen Arztes dessen Rechner mit einem Remote-Rechner gekoppelt ist, der die Auswertung für eine Vielzahl von nur rudimentär in der Arztpraxis vorgehaltenen medizinischen Untersuchungsgeräte herstellt. Die Untersuchungsgeräte sollen nur als abgespeckte Sensorglieder beim lokal niedergelassenen Arzt vorhanden sein, während die teueren Ansteuer- und Auswerteeinrichtungen nur beim zentralen Remote-Rechner vorhanden sind.

Abgesehen davon, dass für die meisten Untersuchungsgeräte, beispielsweise ein Röntgengerät oder einen MR-Scanner, ein solcher vereinfachter Sensorkopf überhaupt nicht sinnvoll denkbar ist, kann dieses System aber bei der der vorliegenden Anmeldung zugrunde liegenden Aufgabenstellung eines vollständig vorhandenen Untersuchungsgeräts beim niedergelassenen - Arzt, der aber für Spezialuntersuchungen nicht genau weiß, wie er dieses Untersuchungsgerät einstellen und bedienen soll, keine Hilfestellung geben.

Der Erfindung liegt daher die Aufgabe zugrunde, eine Vorrichtung zu schaffen, um dieses Wissen von Spezialisten den einfachen Fachkollegen zugänglich zu machen, wobei natürlich auch die Vergütung für die Spezialisten in geeigneter Weise sichergestellt werden muss.

Zur Lösung dieser Aufgabe ist erfindungsgemäß vorgesehen, dass ein Übertragungssystem eines Handhabungsprocederes in Form von Pulssequenzen, Protokollen, Parametersätzen od. dgl. für das Untersuchungsgerät, abgestimmt auf konkrete medizinische Diagnosen des anfragenden Nutzers, mit einem Lizenzabrechnungssystem gekoppelt ist, das dem das Handhabungsprocedere erstellenden Experten eine Lizenzvergütung zukommen lässt, sowie mit einer Sperrvorrichtung, die eine erweiterte Nutzung über die vereinbarte und bezahlte Nutzung hinaus verhindert, wobei bevorzugt ein Direktabbuchungssystem vorgesehen ist, das die Lizenzgebühr mit der Übersendung des Procederes direkt einzieht.

Um Streitigkeiten aufgrund der Abrechnung - unabhängig davon, ob ein Direkteinzug erfolgt oder nur eine In-Rechnung-Stellung, möglichst zu vermeiden bzw. problemlos klären zu können, kann in Weiterbildung der Erfindung vorgesehen sein, dass das Procedere beim abgebenden Experten, dabei kann es sich um einen spezialisierten Facharzt aber auch gegebenenfalls um'ein hochspezialisiertes Expertensystem handeln, und/oder beim empfangenden Nutzer mit Datum, Zeit, Nutzernamen, Patientennamen, sowie gegebenenfalls Lizenzsumme, archiviert wird.

Um die Vorrichtung für den Experten interessant zu halten, das heißt um die Gefahr zu vermeiden, dass der Nutzer für eine einmalige Nutzung eine Lizenzgebühr zahlt und das ihm zur Verfügung gestellte Procedere dann vielfach umsonst weiternutzen kann, kann in Weiterbildung der Erfindung vorgesehen sein, dass das Handhabungsprocedere einen Startzeitpunkt und/oder einen Verfallszeitpunkt enthält, vor bzw. nach dem es nicht benutzbar ist, wobei darüber hinaus auch vorgesehen sein kann, dass das Handhabungsprocedere eine Nutzerbegrenzung auf eine vorgebbare Anzahl von Anwendungen enthält.

Diese Nutzerbegrenzung kann dabei gemäß einem weiteren Merkmal der Erfindung so ausgestaltet sein, dass das Handhabungsprocedere einen Identifikationscode enthält, der die Benutzung auf eines oder mehrere Zielsysteme und/oder speziell definierte Patienten oder Probanten einschränkt. Mit der unverlierbaren Kopplung mit dem Patientennamen, für den das Procedere erstellt bzw. vom Experten ausgewählt worden ist, kann es der Nutzer, also der einfache Fachkollege vor Ort, nur für diesen einen Patienten in seinem Untersuchungsgerät nutzen. Diese Nutzerbegrenzung kann dabei noch dadurch weiter vereinfacht werden, dass das Handhabungsprocedere als Datensatz verschlüsselt ist, der unmittelbar in das Untersuchungsgerät des Nutzers einspielbar ist, um dessen Parametereinstellungen und Untersuchungsabfolgen zu steuern.

Bei der Nutzung der Vorrichtung befragt der Nutzer, insbesondere ein Radiologe, aufgrund einer unklaren Diagnose den Experten bzw. ein hochspezialisiertes Expertensystem, gegebenenfalls unter Vorlage der vorhandenen radiologischen Befunde, wobei der Experte daraufhin ein geeignetes Handhabungsprocedere zur Durchführung weiterer speziell konfigurierter Untersuchungen mit den Untersuchungsgeräten des Nutzers auswählt bzw. neu erstellt und dieses Handhabungsprocedere über das Übertragungssystem bei gleichzeitiger Lizenzabrechnung an den Nutzer übermittelt.

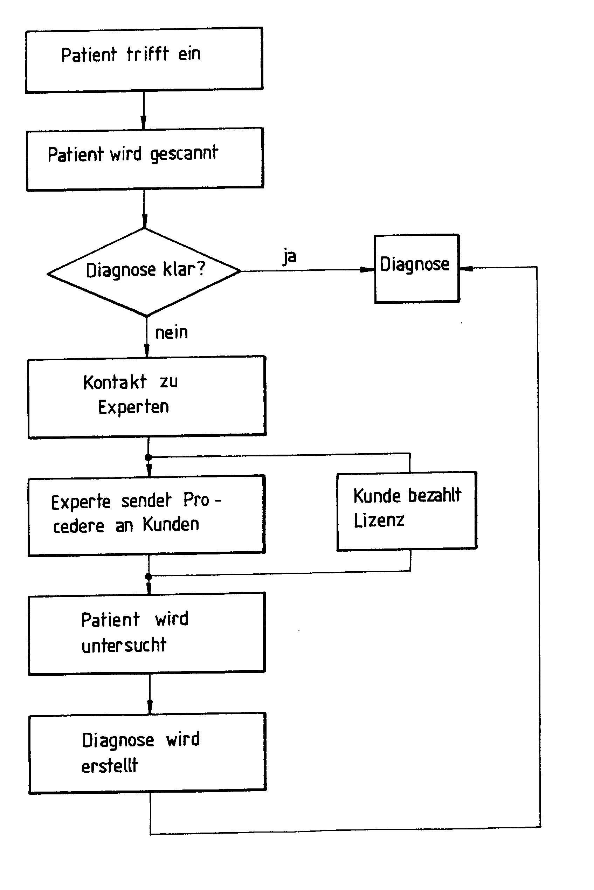

Die Erfindung soll anhand eines in der Zeichnung wiedergegebenen vereinfachten Ablaufdiagramms näher erläutert werden.

Nachdem ein Patient beim Nutzer, beispielsweise einem Radiologen, eingetroffen ist, wird er aufgrund eines Anfangsverdachts durch die Voruntersuchung, entweder des Radiologen oder des überweisenden Hausarztes, gescannt. Ist danach die Diagnose klar, so ist das Verfahren beendet.

Ist die Diagnose aber unklar oder kann der Radiologe aufgrund des Anfangsverdachts des Hausarztes gar keine geeignete Untersuchung durchführen, so kommt das erfindungsgemäße Beratungssystem unter lizenzkontrollierten Bedingungen ins Spiel. Der Nutzer nimmt zu diesem Zweck Kontakt zu einem Experten, insbesondere einem spezialisierten Fachkollegen einer Hochschule, auf und unterbreitet ihm die Anfangsdiagnose gegebenenfalls unter Übermittlung der vorhandenen radiologischen Untersuchungsbefunde. Der Experte sendet daraufhin ein Handhabungsprocedere an den Kunden, das die notwendigen Untersuchungsschritte unter gleichzeitiger Angabe der optimalen Einstellung der Geräteparameter für diese Spezialuntersuchung enthält, was am besten in Form eines Datensatzes geschehen kann, der direkt in die Steuerung des Untersuchungsgerätes des Nutzers eingespielt werden kann. Mit der Übersendung dieses Handhabungsprocederes erfolgt die Rechnungsstellung für die zu zahlende Lizenzgebühr, insbesondere die direkte Abbuchung dieser Lizenzgebühr, von einem zu diesem Zweck eingerichteten Konto des Nutzers, zu dem der Experte über die Vorrichtung Zugriff hat.

Der Patient wird daraufhin beim Nutzer mit dessen medizinischen Untersuchungsgeräten unter Verwendung des Handhabungsprocederes des Experten untersucht und danach die Diagnose erstellt.

Für den seltenen Fall, dass auch jetzt noch Probleme mit der Erstellung einer eindeutigen Diagnose gegeben sind, kann selbstverständlich in einer Schleife nochmals Kontakt zum Experten aufgenommen werden, der unter Verwendung der nunmehr vorliegenden Untersuchungsbefunde sein Handhabungsprocedere abändern oder ergänzen und erneut an den Nutzer übersenden kann.

## Patentansprüche

1. Vorrichtung zur Zugänglichmachung von Expertenwissen für einen Nutzer (Arzt) eines medizinischen Untersuchungsgerätes
**dadurch gekennzeichnet, dass** ein Übertragungssystem eines Handhabungsprocederes in Form von Pulssequenzen, Protokollen, Parametersätzen od. dgl. für das Untersuchungsgerät, abgestimmt auf konkrete medizinische Diagnosen des anfragenden Nutzers, mit einem Lizenzabrechnungssystem gekoppelt ist, das dem das Handhabungsprocedere erstellenden Experten eine Lizenzvergütung zukommen lässt, sowie mit einer Sperrvorrichtung, die eine erweiterte Nutzung über die vereinbarte und bezahlte Nutzung hinaus verhindert.

2. Vorrichtung nach Anspruch 1, **dadurch gekennzeichnet, dass** das Procedere beim abgebenden Experten und/oder beim empfangenden Nutzer mit Datum, Zeit, Nutzername, Patientenname sowie gegebenenfalls Lizenzsumme archiviert wird.

3. Vorrichtung nach Anspruch 1 oder 2, **dadurch gekennzeichnet, dass** das Handhabungsprocedere einen Startzeitpunkt und/oder einen Verfallszeitpunkt enthält, vor bzw. nach dem es nicht benutzbar ist.

4. Vorrichtung nach einem der Ansprüche 1 bis 3, **dadurch gekennzeichnet, dass** das Handhabungsprocedere eine Nutzerbegrenzung auf eine vorgebbare Anzahl von Anwendungen enthält.

5. Vorrichtung nach einem der Ansprüche 1 bis 4, **dadurch gekennzeichnet, dass** das Handhabungsprocedere einen Identifikationscode enthält, der die Benutzung auf eines oder mehrere Zielsysteme und/oder speziell definierte Patienten oder Probanten einschränkt.

6. Vorrichtung nach einem der Ansprüche 1 bis 5, **gekennzeichnet durch** ein Direktabbuchungssystem, das die Lizenzgebühr mit der Übersendung des Procederes direkt einzieht.

7. Vorrichtung nach einem der Ansprüche 1 bis 6, **dadurch gekennzeichnet, dass** das Handhabungsprocedere als Datensatz verschlüsselt ist, der unmittelbar in das Untersuchungsgerät einspielbar ist, um dessen Parametereinstellungen und Untersuchungsabfolgen zu steuern.

8. Verfahren zur Nutzung der Vorrichtung nach einem der Ansprüche 1 bis 7, **dadurch gekennzeichnet, dass** der Nutzer, insbesondere ein Radiologe, aufgrund einer unklaren Diagnose den Experten, gegebenenfalls unter Vorlage der vorhandenen radiologischen Befunde befragt, dass der Experte ein geeignetes Handhabungsprocedere zur Durchführung weiterer, speziell konfigurierter Untersuchungen mit den Geräten des Nutzers auswählt bzw. neu erstellt und dieses Handhabungsprocedere das Übertragungssystem bei gleichzeitiger Lizenzabrechnung an den Nutzer übermittelt.
